Europäisches Patentamt

European Patent Office

Office européen des brevets

(19)

(11)  **EP 1 591 113 A1**

(12)  **EUROPEAN PATENT APPLICATION**
published in accordance with Art. 158(3) EPC

(43) Date of publication:
**02.11.2005  Bulletin 2005/44**

(51) Int Cl.[7]: **A61K 31/122**, A61K 35/78,
A61P 9/00, A61P 9/08,
A61P 9/12, A23L 1/30

(21) Application number: **04702826.1**

(22) Date of filing: **16.01.2004**

(86) International application number:
**PCT/JP2004/000324**

(87) International publication number:
**WO 2004/064818 (05.08.2004 Gazette 2004/32)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **17.01.2003  JP 2003009644
21.11.2003  JP 2003392602**

(71) Applicant: **KIRIN BEER KABUSHIKI KAISHA
Tokyo 104-8288 (JP)**

(72) Inventors:
• **TAKEUCHI, Akihiko, Kirin Beer K. K.
Yokohama-shi, Kanagawa 2300004 (JP)**
• **SATO, Taku, Kirin Beer K. K.
Takasaki-shi, Gunma 3701295 (JP)**
• **YAJIMA, Hiroaki, Kirin Beer K. K.
Yokohama-shi, Kanagawa 2300004 (JP)**
• **MIURA, Yutaka, Kirin Beer K. K.
Yokohama-shi, Kanagawa 2300004 (JP)**
• **KONDO, Keiji, Kirin Beer K. K.
Tokyo 1048288 (JP)**
• **YOSHIDA, Aruto, Kirin Beer K. K.
Yokohama-shi, Kanagawa 2300004 (JP)**
• **YOSHIDA, Kiyoshi, c/o Kawasaki Medical School
Okayama 7010114 (JP)**
• **TOMITA, Junko, c/o Kawasaki Medical School
Kurashiki-shi, Okayama 7010114 (JP)**

(74) Representative: **HOFFMANN EITLE
Patent- und Rechtsanwälte
Arabellastrasse 4
81925 München (DE)**

(54)  **BLOOD PRESSURE-LOWERING AGENT, VASCULAR FLEXIBILITY-IMPROVING AGENT AND
FOODS HAVING THESE FUNCTIONS IMPARTED THERETO**

(57)  According to the present invention, there is provided a blood pressure-lowering agent, a vascular flexibility-ameliorating agent and foods having these functions imparted thereto, which are comprising isohumulones or a hop extract and/or an isomerized hop extract as an active ingredient.

F I G. I

**Description**

BACKGROUND OF THE INVENTION

Field of the Invention

The present invention relates to blood pressure-lowering agents and vascular flexibility-ameliorating agents and foods having these functions imparted thereto.

Background Technology

Hypertension is a symptom that is often observed in everyday life and known to be the lifestyle disease that is associated with the greatest number of patients. Namely, more than 20 million, which is about 20% of the population in Japan, are suffering from hypertension and the number of patients is expected to further increase in the coming aging society as blood pressure usually rises with age.

Further, hypertension is the most significant risk factor for cardiovascular disease, which is the major cause of death in Japan and hypertension can cause stroke, heart failure and renal failure if it is left untreated. On the other hand, it has also been revealed since the large-scale clinical trial, the Veterans Administration Cooperative Study, conducted in 1960s, that development of these fatal diseases can be prevented by controlling blood pressure by drug therapy. Further, Reaven called a group of symptoms including impaired glucose tolerance caused by insulin resistance, hypertension, hyper-VLDL-cholesterolemia, and hypo-HDL-cholesterolemia "Syndrome X," and suggested that amelioration of these symptoms is important in preventing cerebrovascular disorders and coronary artery diseases (Diabetes 37:1595-1607, 1988). Thus, the so-called lifestyle diseases, such as diabetes, hyperlipidemia, and hypertension, tend to converge on a patient and are accordingly called multiple risk factors to cause cerebrovascular disorders and coronary artery diseases.

Hypertension is known to develop vascular endothelial disorders as complications, in which shear stress to the vascular endothelium associated with the increase in blood pressure, body fluid and endocrine disorders, sugar metabolism disorders, and the like are considered to be complicatedly involved. Such vascular endothelial disorders are closely associated with the onset of arteriosclerosis and thus hypertension is suggested to be a possible risk factor for arteriosclerosis. Further, a functional decrease in the vascular endothelium is observed also in hyperlipidemia and diabetes, major risk factors for cardiovascular diseases, or heart failure and thus the improvement or amelioration of the vascular endothelial function is believed to be effective for the prevention of a wide range of cardiovascular diseases. Further, as shown in renal vascular hypertension, it is also revealed that vasodilating agents to ameliorate the vascular endothelial function increase the volume of blood flow and are effective for the purpose of accelerating blood flow (N. Engl. J. Med. 346:1954-1962 (2002)).

In recent years, owing to the remarkable development of measuring instruments, vascular endothelial function has been increasingly assessed in a non-invasive manner by blood flow-dependent flow-mediated dilatation (FMD). It is understood that in this method the vascular endothelial function is assessed by measuring vascular flexibility and elasticity. FMD is dependent on nitric oxide (NO) and is augmented by an increase in the amount of nitric oxide generated by the activity of endothelial NO synthase (eNOS) in vascular endothelial cells. This is because the nitric oxide generated from the vascular endothelial cells acts on vascular smooth muscle cells to relax the blood vessels. On the other hand, FMD diminishes when the extracellular calcium or sodium concentration is reduced (Am. J. Physiol. Heart Circ. Physiol. 282:H1-H5 (2002)).

The mechanism of hypertension development has been gradually revealed with the progress of molecular biology. However, since hypertension is a multi-factor disease that is developed by a wide variety of causative factors which complicatedly interact with each other, various approaches are also necessary for the treatment. Today, two types of therapies, drug therapy and non-drug therapy, are generally applicable for the treatment of hypertension.

Examples of drugs used in the drug therapy include diuretics, β-blockers, calcium channel antagonists, ACE inhibitors, and α-blockers. In drug therapy, quality of life (QOL) of individual patients has to be taken into consideration since the treatment basically lasts for a long period of time. Further, since blood pressure-lowering agents tend to cause circulatory insufficiency in organs due to excessively reduced blood pressure and to generate side effects onto the metabolic system, the extent of pressure reduction has to be appropriate and carefully monitored in their use. Accordingly, it is desirable to find a component having such an effect from materials which have been known to be edible, also from a safety point of view.

On the other hand, non-drug therapy is a fundamental treatment modality for hypertension, which effectively makes us realize that hypertension is treated by solving daily life problems. Further, non-drug therapy is effective also because drugs are foreign to the body so that side effects have to be kept in mind upon their therapeutic use. In non-drug therapy, lifestyle improvement has been proposed. Examples of non-drug therapy generally carried out include reducing weight in obese patients, limiting alcohol consumption, exercising, reducing sodium intake, quitting smoking, and reducing saturated fatty acids and cholesterols in diet. Therapy by everyday meals (dietary therapy) is believed to be of particular importance. Accordingly, development of prominent foods having a blood pressure lowering effect has been strongly desired.

The hop (Humulus lupulus) is a native European perennial which belongs to the family Cannabaceae, and its fruiting bodies (strobili of female flowers), generally called hops, are widely known to be used for adding a bitter taste and aroma to beer and thus have long been ingested by humans. Such bitter taste and aroma come from hop lupulin (yellow granules formed in the root of the inner scales of strobili). Hops are used also as a folk medicine and known to have various physiological effects, such as inducing sadation, encouraging sleep, inducing sound sleep, stimulating appetite, soothing the stomach, and diuretic effect. Further, their anti-diabetic effect has been also reported (Japanese Laid-Open Publication No.70512/1975, Japanese Laid-Open Publication No.59623/1979). Also, in recent years, it has been reported that polyphenols derived from hop scales obtained from hop corns, from which the lupulin part is removed, have activities such as inhibiting lipase activity and suppressing body weight gain (Japanese Laid-Open Publication No.321166/2001, Japanese Laid-Open Publication No. 131080/2001). However, as for humulones and isohumulones that are bitter components of hops, their blood lowering activity has not been known.

SUMMARY OF THE INVENTION

The present inventors have found that isohumulones that are isomerized compounds of humulones, major bitter taste components of hops, have a function to lower blood pressure and a function to improve vascular flexibility. The present invention is based on these findings.

An objective of the present invention is to provide a blood pressure-lowering agent and a vascular flexibility-ameliorating agent and foods having these functions imparted thereto.

According to the present invention, there is provided a composition for lowering blood pressure, comprising isohumulones or a hop extract and/or an isomerized hop extract as an active ingredient.

Further, according to the present invention, there is provided a composition for preventing, treating or ameliorating hypertension, comprising isohumulones, or a hop extract and/or an isomerized hop extract as an active ingredient.

According to the present invention, there is further provided a composition for improving or ameliorating vascular flexibility, comprising isohumulones, or a hop extract and/or an isomerized hop extract as an active ingredient.

According to the present invention, there is furthermore provided a composition for improving or ameliorating vascular endothelial function, comprising isohumulones, or a hop extract and/or an isomerized hop extract as an active ingredient.

According to the present invention, there is provided a composition for vascular dilatation or blood flow acceleration, comprising isohumulones, or a hop extract and/or an isomerized hop extract as an active ingredient.

According to the present invention, there is furthermore provided a food for use in lowering blood pressure, preventing, treating or ameliorating hypertension, improving or ameliorating vascular flexibility, improving or ameliorating vascular endothelial function, or in vascular dilatation or blood flow acceleration, comprising isohumulones, or a hop extract and/or an isomerized hop extract.

The treatment of hypertension or cardiovascular diseases with drugs often requires a long period of time and various problems such as side effects due to increased dosages and prolonged administration cannot be ignored. Isohumulones, or a hop extract and/or an isomerized hop extract contained in the compositions and foods according to the present invention are derived from hops that have been used as a food for many years. Therefore, compositions and foods according to the present invention are advantageous in that they have little side effects and highly safe when taken by a patient over a long period of time. In particular, the compositions and foods according to the present invention have an excellent characteristic that they advantageously have a function to prevent liver disorders as mentioned hereinafter while some conventional blood pressure-lowering agents disadvantageously cause liver disorders as a side effect.

BRIEF DESCRIPTION OF THE DRAWINGS

Figure 1 shows the action of isohumulones (IHus) a vehicle (Veh) on the 80 mM KCl contraction in Example 4. The isohumulones relaxed the specimen of rat aorta contracted with 80 mM KCl in a manner of concentration-dependence.

Figure 2 shows the action of isohumulones (IHus) and a vehicle on the phenylephrine ($10^{-6}$ M) contraction in Example 4. The isohumulones relaxed the specimen of rat aorta contracted with phenylephrine ($10^{-6}$ M) in a manner of concentration-dependence.

Figure 3 shows the action of isohumulones (IHus) on the 80 mM KCl contraction (open circles) and the phenyle-phrine ($10^{-6}$ M) contraction (closed circles) in Example 4.

Figure 4 shows the improvement of vascular endothelial function by a hop extract in Example 5. "HE" represents the group administered with the hop extract and "placebo" represents the group administered with placebo. For the group administered with the hop extract, a statistical significant test was carried out using a t-test (Excel 200, Microsoft) all against the value on week zero. In the figure, * represents a significance level of 1% or less.

Figure 5 shows the improvement of vascular endothelial function by a hop extract in Example 5. F, H and J are the subjects having deteriorated vascular endothelial function.

## DETAILED DESCRIPTION OF THE INVENTION

### Active ingredients and methods for their production

Isohumulones contained in the compositions and foods according to the present invention can preferably be selected from the group consisting of isohumulone, isoadhumulone, isocohumulone and combinations thereof.

The isohumulones can be those commercially available. An active ingredient according to the present invention can also be produced according to known methods; for example, they can be synthesized by the method described in Developments in Food Science 27, CHEMISTRY AND ANALYSIS OF HOP AND BEER BITTER ACIDS, M. Verzele, ELSEVIER. An active ingredient according to the present invention can be obtained by isolating and purifying a hop extract or an isomerized hop extract obtained according to the methods described hereinafter.

Extract derived from hop lupulin can be used as a hop extract contained in compositions and foods according to the present invention. An isomerized hop extract contained in compositions and foods according to the present invention can be obtained by positively isomerizing the extract derived from hop lupulin. The hop is a perennial plant which belongs to the family Cannabaceae, and hops are its strobili (matured unpollinated female flowers). Hop lupulin is a raw material for beer brewing and is used to impart bitter taste and aroma to the beer. Further, in the beer brewing process, humulones (e.g., humulone, cohumulone, adhumulone, posthumulone, and prehumulone) are isomerized to isohumulones (e.g., isohumulone, isocohumulone, isoadhumulone, isoposthumulone, and isoprehumulone) to impart characteristic taste and aroma to the beer.

A hop extract can be prepared by subjecting strobili or pressed product thereof, as it is or after crushing, to an extraction process. The extraction can be carried out, for example, by a method used for the preparation of hop extract for the beer brewing, such as the extraction method using ethanol solvent and the supercritical carbon dioxide extraction method. In particular, the supercritical carbon dioxide extraction is characterized in that the resulting product contains a low concentration of polyphenol component and bitter component and essential oil component are highly concentrated. Further, hop extraction can be carried out using other generally used methods, including a method in which hop strobili, crushed products thereof, or the like are submersed in a cold or warmed solvent; a method in which extraction is carried out with heating and stirring and then the resulting extract is obtained by filtration; and a percolation method. After removing solids by filtration or centrifugation if necessary, the resulting extract can be used as it is or after removing the solvent by distillation and partially concentrating or drying, depending on the mode of use. Further, after concentrating or drying, the extract can be washed and purified with an insoluble solvent or further dissolved and suspended in an appropriate solvent for use. Further in the present invention, for example, the solvent extract obtained as described above can be dried using general means such as drying under the reduced pressure and freeze drying to obtain a dried hop extract for use.

Examples of solvents to be used for the above-mentioned extraction include water; lower alcohols having one to four carbon atoms, such as methanol, ethanol, propanol and butanol; lower alkyl esters such as ethyl acetate ester; glycols such as ethylene glycol, butylene glycol, propylene glycol, and glycerin; other polar solvents such as ethyl ether, acetone, and acetic acid; hydrocarbons such as benzene and hexane; non-polar solvent such as ethers, e.g., ethyl ethers and petroleum ethers, or known organic solvents. These solvents can be used alone or in combination of two or more kinds.

Further, if necessary, insolubles can be removed by filtration or the solvent can be dried to solid by concentrating extract, for example, under the reduced pressure. Further, preferably, crushed strobili are subjected to the supercritical carbon dioxide extraction or the liquid carbon dioxide gas extraction.

The crude extracts extracted as above contain humulones and additionally their isomerized products isohumulones; however, it is preferred to further isomerize these crude extracts by heating in the presence of alkali or magnesium oxide so as to obtain a high content of isohumulones. By the isomerization, humulones in the hop extracts are completely converted into isohumulones.

The extracts thus obtained can be used as they are for pharmaceutical preparations; however, it is preferable to use a fraction containing active ingredients at higher concentrations. Hop extracts extracted by various methods and isomerized extracts are commercially available as a beer additive and can be used in compositions and foods according to the present invention. Examples of the usable products include a hop extract in which humulones and lupulones are primarily extracted from crushed hop strobili using the supercritical carbon dioxide extraction method (e. g., CO2 Pure Resin Extract (Hopsteiner)), an isomerized carbon dioxide extract of crushed hop strobili (e.g., Isomerized Kettle Extract (SS. Steiner) mainly consisting of isohumulones and lupulones), and a water soluble extract in which carbon dioxide extract of crushed hop strobili is isomerized and then converted into a potassium salt to obtain a low viscous fluid (e.g., ISOHOPCO2N (English Hop Products) and ISOHOP R (Botanix) primarily consisting of isohumulones).

Further, it should be understood that these extracts can be further concentrated to fractions containing highly concentrated active ingredients by using the above-mentioned methods or the like.

Use

An increase in intracellular calcium ion concentration is important for vascular contraction. It is believed that vascular contraction by a high concentration of potassium is caused primarily by the opening of membrane voltage-dependent calcium channels by depolarization of vascular smooth muscle, which leads to extracellular calcium influx into the cells to increase the intracellular calcium concentration. On the other hand, phenylephrine-induced vascular contraction is believed to be caused primarily by an increase in the intracellular $Ca^{2+}$ concentration generated by $Ca^{2+}$ influx through receptor-operative Ca channels and $Ca^{2+}$ release from sarcoplasmic reticulum induced by $IP_3$ production.

Isohumulones relaxed both the contraction induced by a high concentration of potassium and the contraction induced by a high concentration of phenylephrine in dissected rat blood vessels (see Example 4). Accordingly, it can be suggested that the isohumulones exhibit the vascular relaxing action by suppressing a contraction process common to both contractions (decrease in intracellular $Ca^{2+}$, decrease in $Ca^{2+}$ sensitivity, or the like). Further, since the relaxing action by isohumulones tends to be more strongly exhibited in the contraction induced by a high concentration of potassium than in the phenylephrine-induced contraction, it can also be suggested that isohumulones exhibit the vascular relaxing action by suppressing membrane voltage-dependent Ca channels. Further, as described below, isohumulones augment flow-mediated dilatation (FMD) to increase the volume of blood flow (see Example 5).

Accordingly, isohumulones, or a hop extract and/or an isomerized hop extract can be used for the purpose of vascular dilatation and the blood flow acceleration. The vascular dilatation and blood flow acceleration are known to reduce or relieve sensitivity to cold due to poor circulation, stiff shoulders, back pain, swelling of the limbs, and fatigue. Accordingly, it will be understood by those skilled in the art that use in such occasions is also within the scope of the present invention.

Further, a tendency to lower blood pressure was observed when a single dose of isohumulones purified from isomelized hop extract was given to spontaneously hypertensive model animals, i.e., spontaneously hypertensive rats (SHRs) (see Example 3). Further, a decrease in blood pressure was observed when an isomerized hop extract was administered to humans with slightly high blood pressure (see Example 1).

Accordingly, isohumulones, or a hop extract and/or an isomerized hop extract can be used for the purpose of lowering blood pressure. Isohumulones, or a hop extract and/or an isomerized hop extract can also be used for preventing, treating or ameliorating hypertension.

When an isomerized hop extract was administered to humans over a long period of time, it was observed that along with a decrease in blood pressure, decreases were observed entirely in GOT (glutamic oxaloacetic transaminase), GPT (glutamic pyruvic transaminase), γ-GTP (glutamyl transpeptidase), and LDH (lactate dehydrogenase) in the blood. GOT and GPT leak into the blood stream from damaged liver cells to increase their levels when the liver is affected by some reasons, and thus their levels are known to be high in patients with fatty liver or alcoholic liver disorder. γ-GTP and LDH are also known to be indices in a similar way. Generally in therapy with drugs including conventional blood lowering agents, there are cases where the risk of liver disorders is concerned as a side effect; however, when active ingredients according to the present invention are used for the prevention, treatment or amelioration of blood pressure increase or the amelioration or improvement of vascular endothelial function, they exhibit an excellent preventive as well as therapeutic effect on liver disorders. Accordingly, compositions and foods according to the present invention are advantageous in that they have preventive and therapeutic effects on liver disorders. Further, isohumulones, or a hop extract and/or an isomerized hop extract can be used for the prevention, treatment or amelioration of liver disorders.

Isohumulones significantly augmented FMD from immediately after administration to humans (see Example 5). From this fact, it can be suggested that isohumulones increase the amount of nitric oxide generated from vascular endothelial cells. Further, since this effect is observed not only in humans with normal vascular endothelial function but also in humans with decreased vascular endothelial function (see Example 5), isohumulones, or a hop extract and/or an isomerized hop extract are expected to have both improving and ameliorating effects on vascular endothelial function. Further, since FMD is an index to assess blood vessel flexibility, isohumulones, or a hop extract and/or an isomerized hop extract are expected to have both improving and ameliorating effects on the flexibility of blood vessels.

Accordingly, isohumulones, or a hop extract and/or an isomerized hop extract can be used for the purpose of improving and/or ameliorating blood vessel flexibility. Isohumulones, or a hop extract and/or an isomerized hop extract can also be used for the purpose of improving and/or ameliorating vascular endothelial function. Further, isohumulones, or a hop extract and/or an isomerized hop extract can be used for the treatment, prevention, or amelioration of vascular diseases caused by the deterioration of vascular endothelial function, such as arteriosclerosis.

The extent of the improvement and amelioration of blood vessel flexibility or vascular endothelial function can be assessed using FMD as an index. Since FMD is an index to assess the blood vessel flexibility, it can be said that the greater the FMD value, the higher the blood vessel flexibility and the smaller the FMD value the insufficient the blood vessel flexibility. The FMD value of healthy subjects measured by this method is about 6 or more and the FMD value of patients with and candidates for blood vessel diseases such as arteriosclerosis is smaller than that. The FMD value of patients with and candidates for blood vessel diseases such as arteriosclerosis is generally said to be less than 4.

In the present invention, the terms "improvement" and "amelioration" in blood vessel flexibility or vascular endothelial function are to be used individually for healthy subjects and patients with and candidates for blood vessel diseases such as arteriosclerosis. For example, when the FMD value of a healthy individual is increased, the blood vessel flexibility and the vascular endothelial function of such individual are judged to be improved. Further, when the FMD value of a patient with or a candidate for vascular diseases such as arteriosclerosis is increased, the blood vessel flexibility and the vascular endothelial function of such individual are judged to be ameliorated.

According to the present invention, there is provided a method for lowering blood pressure, comprising administering a therapeutically or prophylactically effective amount of isohumulones or a hop extract and/or an isomerized hop extract, to a mammal.

According to the present invention, there is provided a method for preventing, treating or ameliorating hypertension, comprising administering a therapeutically or prophylactically effective amount of isohumulones or a hop extract and/or an isomerized hop extract, to a mammal.

According to the present invention, there is provided a method for improving or ameliorating blood vessel flexibility, comprising administering a therapeutically or prophylactically effective amount of isohumulones or a hop extract and/or an isomerized hop extract, to a mammal.

According to the present invention, there is provided a method for improving or ameliorating vascular endothelial function, comprising administering a therapeutically or prophylactically effective amount of isohumulones or a hop extract and/or an isomerized hop extract, to a mammal.

According to the present invention, there is provided a method for dilating blood vessels or accelerating blood flow, comprising administering a therapeutically or prophylactically effective amount of isohumulones or a hop extract and/or an isomerized hop extract, to a mammal.

Further, according to the present invention, there is provided use of isohumulones or a hop extract and/or an isomerized hop extract for the manufacture of a composition for lowering blood pressure.

According to the present invention, there is provided use of isohumulones or a hop extract and/or an isomerized hop extract for the manufacture of a composition for preventing, treating or ameliorating hypertension.

According to the present invention, there is provided use of isohumulones or a hop extract and/or an isomerized hop extract for the manufacture of a composition for improving or ameliorating blood vessel flexibility.

According to the present invention, there is provided use of isohumulones or a hop extract and/or an isomerized hop extract for the manufacture of a composition for improving or ameliorating vascular endothelial function.

According to the present invention, there is provided use of isohumulones or a hop extract and/or an isomerized hop extract for the manufacture of a composition for dilating blood vessels or accelerating blood flow.

<u>Composition and food</u>

When a composition according to the present invention is provided as a pharmaceutical composition, it can be produced by mixing isohumulones or a hop extract and/or an isomerized hop extract as an active ingredient with pharmaceutically acceptable additives. A pharmaceutical composition according to the present invention can be administered orally or non-orally. Examples of oral formulations include granules, dispersible powders, tablets (including sugar-coated tablets), pills, capsules, syrups, emulsions, and suspensions. Examples of non-oral formulations include injections (e.g., subcutaneous injections, intravenous injections, intramuscular injections, peritoneal injections), intravenous drips, preparations for external use (e.g., nasal formulations, percutaneous agents, ointments), and suppositories (e.g., rectal suppositories, vaginal suppositories). These pharmaceutical preparations can be formulated by a method generally used in this field using pharmaceutically acceptable carriers. Examples of pharmaceutically acceptable carriers include excipients, binding agents, diluents, additives, flavoring agents, buffers, thickening agents, coloring agents, stabilizers, emulsifying agents, dispersing agents, suspending agents, and preservatives; for example, magnesium carbonate, magnesium stearate, talc, sucrose, lactose, pectin, dextrin, starch, gelatin, tragacanth, methylcellulose, sodium carboxymethylcellulose, low-melting wax, and cacao butter can be used as a carrier.

Pharmaceutical preparations can be produced, for example, as follows.

Oral formulations can be produced by adding excipients (e.g., lactose, sucrose, starch, mannitol), disintegrating agents (e.g., calcium carbonate, calcium carboxymethylcellulose), binding agents (e.g., pregelatinized starch, gum arabic, carboxymethylcellulose, polyvinylpyrrolidone, hydroxypropylcellulose), lubricating agents (e.g., talc, magnesium stearate, polyethylene glycol 6000), and the like to an active ingredient, pressing the admixture into an appropriate form, and if necessary, coating for taste masking, enteric film coating or durability using a known method. Examples of coating agents to be used include ethylcellulose, hydroxymethylcellulose, polyoxyethylene glycol, cellulose acetate phthalate, hydroxypropylmethylcellulose phthalate, and Eudragit (methacrylic acid-acrylic acid copolymer; Roehm, Germany).

Formulations for injection can be produced by dissolving, suspending or emulsifying an active ingredient in an aqueous solvent (e.g., distilled water, physiological saline, Ringer's solution) or an oily medium (e.g., vegetable oils such as olive oil, sesame oil, cotton seed oil, and corn oil, or propylene glycol) together with dispersing agents (e.g., Tween 80 (Atlas Powder, USA), HCO 60 (Nikko Chemicals), polyethylene glycol, carboxymethylcellulose, sodium alginate), preservatives (e.g., methylparabene, propylparabene, benzylalcohol, chlorobutanol, phenol), osmosis equilibrating agents (e.g., sodium chloride, glycerin, sorbitol, glucose, invert sugar) and the like. If desired, additives such as solubilizing agents (e.g., sodium salicylate, sodium acetate), stabilizing agents (e.g., human serum albumin) and analgesic agents (e.g., benzalkonium chloride, procaine hydrochloride) may be added.

Pharmaceutical preparations for external use can be produced by formulating an active ingredient into a solid, semi-solid or liquid composition. For example, the above-mentioned solid composition can be produced by formulating an active ingredient into a powder as it is or by mixing with addition of excipients (e.g., lactose, mannitol, starch, microcrystal cellulose, sucrose), thickening agents (e.g., natural gums, cellulose derivatives, acrylic acid polymers) and the like to the active ingredient. The above-mentioned liquid composition can be produced in almost the same manner as described for injectable preparations. The semi-solid composition is preferably an aqueous or oleaginous gel or ointment. Further, any of these compositions can contain a pH controlling agent (e.g., carbonic acid, phosphoric acid, citric acid, hydrochloric acid, sodium hydroxide), a preservative (e.g., paraoxybenzoic acid esters, chlorobutanol, benzalkonium chloride) and the like. Suppositories can be produced by formulating an active ingredient into an oleaginous or aqueous solid, semi-solid, or liquid composition. Examples of the oleaginous base to be used for such compositions include higher fatty acid glycerides (e.g., cacao oil, Witepsols (Dynamite Nobel)), medium fatty acids (e.g., Miglyols (Dynamite Nobel)), and vegetable oils (e.g., sesame oil, soybean oil, cotton seed oil). Examples of the aqueous base include polyethylene glycols and propylene glycols. Further, examples of the aqueous gel base include natural gums, cellulose derivatives, vinyl polymers, and acrylic acid polymers.

Foods according to the present invention are foods and drinks containing an effective amount of isohumulones, or a hop extract and/or an isomerized hop extract. The expression "containing an effective amount of isohumulones, or a hop extract and/or an isomerized hop extract" herein means that isohumulones, or a hop extract and/or an isomerized hop extract are contained in such an amount that they can be ingested in the range described below when each of the foods and drinks is taken in an ordinary amount. Isohumulones, or a hop extract and/or an isomerized hop extract can be blended into foods according to the present invention, as it is or in forms of the above-mentioned compositions. More specifically, foods according to the present invention can be those prepared as foods or drinks by using at least one of isohumulones, or a hop extract and/or an isomerized hop extract or the above-mentioned crushed hops or their extract, as they are, those further being admixed with various proteins, sugars, fats, trace elements, vitamins, and the like, those being formulated into a form of liquid, semi-liquid, or solid, or those being added to general foods or drinks.

The term "foods" used in the present invention includes health foods, functional foods, foods for specified health use, and foods for patients.

Further, the form of "foods" is not particularly limited and can be, for example, a drink form.

Isohumulones, or a hop extract and/or an isomerized hop extract have a function to lower blood pressure. Accordingly, it is possible to provide foods which simultaneously function in preventing and ameliorating hypertension and preventing hypertensive preconditions from developing hypertension, by blending an active ingredient according to the present invention into daily foods, health foods and functional foods taken as supplements, suitably foods containing salt, and the like. Namely, foods according to the present invention can be provided as foods appropriate for consumers having relatively high blood pressure, in particular as foods for specified health use.

Further, isohumulones, or a hop extract and/or an isomerized hop extract have functions to improve and ameliorate blood vessel flexibility, improve and ameliorate vascular endothelial function, dilate blood vessels and accelerate blood flow. Accordingly, it is possible to provide foods which simultaneously function in preventing and ameliorating hypertension and preventing the development of blood vessel diseases caused by deterioration of blood vessel flexibility and deterioration of vascular endothelial function, such as arteriosclerosis, preventing arteriosclerotic preconditions from developing arteriosclerosis, and accelerating blood flow, by blending an active ingredient according to the present invention into daily foods, health foods and functional foods taken as supplements, suitably foods containing fats and sugars, and the like. Namely, foods according to the present invention can be provided as foods appropriate for consumers having a high risk of cardiovascular diseases, such as those with a high blood lipid or sugar level, in particular as foods for specified health use.

Examples of such foods and drinks include, but not particularly limited to, those containing carbohydrate such as rice products, noodles, breads, and pastas; various confectionaries including western sweets such as cookies and cakes, Japanese sweets such as buns with a filling and steamed adzuki-bean pastes, candies, chewing gums, and chilled sweets such as yogurt and puddings; alcohol level such as whiskies, bourbons, spirits, liqueurs, wines, fruit liquors, sake, Chinese liquors, shochu, beers, non-alcoholic beers with an alcohol level of not more than 1%, foaming wines, and clear liquors; non-alcoholic drinks such as drinks with fruit juice, drinks with vegetable juice, drinks with fruit juice and vegetable juice, soft drinks, cow's milk, soymilk, milk drinks, drink-type yogurt, coffee, cocoa, tea drinks, nutritional drinks, sports drinks, and mineral water; processed foods with eggs; and processed foods (including delicacies) using seafood (e.g., squid, octopus, shellfish, eel) or meat (including entails such as liver).

Examples of tea drinks include black tea, green tea, roasted barley tea, tea with popped and roasted rice, sencha, gyokuro, roasted tea, oolong tea, turmeric tea, pu-erh tea, rooibos tea, rose tea, twig tea, and herbal tea (e. g., mint tea, jasmine tea).

Examples of fruits used in drinks with fruit juice and drinks with fruit juice and vegetable juice include apples, oranges, grapes, bananas, pears and Japanese apricots. Further, examples of vegetables used in drinks with vegetable juice and drinks with fruit juice and vegetable juice include tomatoes, carrots, celery, cucumbers, and watermelons.

Further, isohumulones, or a hop extract and/or an isomerized hop extract also have a function to prevent liver disorders as mentioned above; therefore, examples of preferred embodiments by which this advantage is further positively realized include foods and drinks containing virtually no alcohol, namely non-alcohol drinks, in a sense to limit alcohol intake.

When isohumulones, or a hop extract and/or an isomerized hop extract or crushed hop or hop extract are used as a food product by admixing with a general food material, it is desirable to prevent the food or drink from being affected by hop bitterness by limiting the amount of use or manipulatively masking.

Since the compositions and foods according to the present invention use hop extract components or their derivatives that have been ingested by humans for many years as foods or drinks, they are low in toxicity and can be used safely for mammals (e.g., humans, mice, rats, rabbits, canines, cats, cattle, horses, pigs, monkeys). The amount of administration or intake for an active ingredient according to the present invention can be determined depending on the recipient, recipient's age, body weight, and symptoms, the time of administration, the type of dosage form, the route of administration, the combination with other medicines, and the like. For example, an active ingredient according to the present invention can be administered as a medicine to an adult orally at a dose ranging from 0.5 to 100 mg/kg body weight, preferably 1 to 50 mg/kg body weight, and non-orally at a dose ranging from 0.05 to 50 mg/kg body weight, preferably 0.5 to 50 mg/kg body weight, in a single dose or in 2 or 3 divided doses daily. Appropriate dosages of medicines having other functions to be used in combination with an active ingredient according to the present invention can be determined based on their individual dosages for clinical use. Further, when taken as foods, isohumulones, or a hop extract and/or an isomerized hop extract can be admixed in the foods so that the amount of its daily ingestion for an adult ranges from 30 to 6000 mg on the basis of an amount of isohumulones, preferably from 60 to 3000 mg on the basis of an amount of isohumulones.

According to the present invention, there is further provided a non-alcohol drink containing isohumulones, or a hop extract and/or an isomerized hop extract, which provides isohumulones, or a hop extract and/or an isomerized hop extract daily in the range from 30 to 6000 mg, preferably from 60 to 3000 mg, on the basis of an amount of isohumulones. This non-alcohol drink is advantageous in that it has effects to lower blood pressure, improve and ameliorate vascular endothelial function and prevent liver disorder. The non-alcohol drinks can preferably be a tea drink.

EXAMPLE

The present invention is further illustrated by the following examples that are not intended as a limitation of the invention.

Reference Example 1: Preparation of isomerized hop extract and isohumulones

An isomerized hop extract of isohumulones (product name: ISOHOP R (Botanix), referred to as the "hop extract" in Examples hereinafter), which was obtained by extracting humulones from hop strobili, isomerizing the humulones into isohumulones and then converting them into a potassium salt was used in the following Examples. This hop extract contains almost no humulones or lupulones but it contains about 30% (w/v) isohumulones.

The hop extract was neutralized with hydrochloric acid and freeze-dried and then 3.5 g of the freeze-dried product were fractionated by silica gel column chromatography (3.5 cm x 33 cm). The column was equilibrated and then eluted with hexane/ethyl acetate (2:1). The resulting eluate was dried under the reduced pressure and the dried product was weighed and then dissolved in ethanol. The purity of the eluate was confirmed by HPLC. Conditions used were a mobile phase of 85% methanol and 15% of 1 %-formic acid aqueous solution, a column of YMC-ODS-AQ 25x250 mm, and a flow rate of 20 ml/min. The isohumulones obtained contained 48% isohumulone, 37% isocohumulone, and 15% isoadhumulone.

Reference Example 2: Preparation of capsules

After adjusting the pH to 7.8 with lactic acid, the hop extract was freeze-dried. Two kinds of capsules were prepared, one for the placebo group and one for the hop extract group. Capsules for the hop extract group were prepared by admixing the above-mentioned freeze-dried hop extract with cornstarch and sucrose fatty acid ester and carrying out filling using a capsule filler. On the other hand, capsules for the placebo group were prepared by admixing cornstarch with sucrose fatty acid ester and then carrying out filling using a capsule filler. Each capsule for the hop extract group contains 100 mg of freeze-dried hop extract, 185 mg of cornstarch, and 5 mg of sucrose fatty acid ester and each capsule for the placebo group contains 250 mg of cornstarch and 5 mg of sucrose fatty acid ester.

Example 1: Effect of hop extract on human subjects

Twenty males and females at the age of 43 to 65 were selected as subjects. The subjects selected are those who have a systolic blood pressure of 103 to 158 mmHg, a fasting blood sugar level of 110 to 146 mg/dl, an HbA1 C of 6.4 to 8.0%, and a BMI of 24 to 34. 4, are capable of keeping their record of life by themselves, and submitted an agreement paper with a seal or signature and a date to agree to participate in this experiment prior to the start of the experiment in view of the ethical consideration.

In the experiment, the capsules for the hop extract group described in Reference Example 2 were used for the hop extract group, and the capsules for the placebo group described in Reference Example 2 were used for the placebo group. One capsule each was given twice a day at different times to both the hop extract group and the placebo group. For example, two capsules were given daily at two different times, after lunch and before going to bed. The total period of administration was 12 weeks and blood sampling and blood pressure measurement were performed in the next mornings of the starting day, week 4, week 8, and week 12 after overnight fasting (fasting for 10 hours or more). All the sampling and measurement were performed in the presence of a physician. During the 12 weeks of the experimental period, accompanying symptoms were not particularly observed. Measurements performed at the time of blood sampling were body weight, systolic blood pressure, diastolic blood pressure, GOT, GPT, $\gamma$-GTP, and LDH. Blood analysis was carried out using an automatic blood analyzer. Data were expressed as average $\pm$ SD. Statistical analyses were performed using Student's paired t-test (Stat View, HeuLinks).

As shown in Table 1, as for body weight, there was no change in the placebo group, whereas a significant decrease was observed in the hop extract group after 8 weeks of ingestion (in Examples hereinafter, a statistical significance test was carried out against the value on week zero. In the Table, * represents a significance level of less than 5% and ** represents a significance level of less than 1 %).

TABLE 1

| Body weight | kg | | | |
|---|---|---|---|---|
| Ingestion (weeks) | 0 | 4 | 8 | 12 |
| Placebo group | 68.08 ± 10.53 | 68.12 ± 10.36 | 67.91 ± 10.4 | 67.66 ± 10.25 |
| Hop extract group | 70.22 ±11.65 | 67.87 ± 11.48 | 69.36 ± 11.07* | 69.95 ± 11.45 |

As shown in Table 2, as for the systolic blood pressure, there was no change in the placebo group entirely after 4 weeks, 8 weeks, and 12 weeks of ingestion as compared to that on week 0, whereas a decrease with time was observed in the hop extract group and the differences were all significant.

TABLE 2

| Systolic blood pressure | mmHg | | | |
|---|---|---|---|---|
| Ingestion (weeks) | 0 | 4 | 8 | 12 |
| Placebo group | 129 ± 17.29 | 128.8 ± 18.79 | 127.5 ± 16.13 | 127.5 ± 16.28 |

TABLE 2 (continued)

| Systolic blood pressure | mmHg | | | |
|---|---|---|---|---|
| Hop extract group | 137.1 ± 14.39 | 130.8 ± 15.58* | 128.6 ± 16.34* | 121.8 ± 13.39** |

As shown in Table 3, as for diastolic blood pressure, a significant change was not observed in either the placebo group or the hop extract group.

TABLE 3

| Diastolic blood pressure | mmHg | | | |
|---|---|---|---|---|
| Ingestion (weeks) | 0 | 4 | 8 | 12 |
| Placebo group | 81.1 ± 9.72 | 78.1 ± 7.72 | 80.6 ± 8.58 | 80.9 ± 9.11 |
| Hop extract group | 84.6 ± 12.02 | 81.5 ± 10.01 | 83.8 ± 9.68 | 80.6 ± 8.33 |

As shown in Table 4, as for GOT, a tendency of increase was observed in the placebo group, whereas a decrease was observed in the hop extract group and the difference was significant after 8 weeks of ingestion.

TABLE 4

| GOT | IU/l | | | |
|---|---|---|---|---|
| Ingestion (weeks) | 0 | 4 | 8 | 12 |
| Placebo group | 25.7 ± 10.98 | 25.5 ± 10.97 | 27 ± 16.9 | 25.8 ±14.02 |
| Hop extract group | 28.6 ± 13.76 | 27.3 ± 9.99 | 22.5 ± 6.59* | 23.6 ± 6.98 |

As shown in Table 5, as for GPT, no change was observed in the placebo group, whereas a tendency of decrease was observed in the hop extract group and the decrease was significant after 8 weeks of ingestion.

TABLE 5

| GPT | IU/l | | | |
|---|---|---|---|---|
| Ingestion (weeks) | 0 | 4 | 8 | 12 |
| Placebo group | 25.4 ± 18.82 | 24.1 ± 17.55 | 24.8 ± 23.89 | 23.8 ±19.13 |
| Hop extract group | 40.8 ± 27.03 | 35.6 ± 20.54 | 27.2 ± 11.8* | 29.3 ± 12.28 |

As shown in Table 6, as for γ-GTP, a tendency of increase was observed in the placebo group, whereas a tendency of decrease was observed in the hop extract group and the difference was significant after 8 weeks of ingestion.

TABLE 6

| γ-GTP | IU/l | | | |
|---|---|---|---|---|
| Ingestion (weeks) | 0 | 4 | 8 | 12 |
| Placebo group | 75.3 ± 96.19 | 70.7 ± 92.69 | 78.3 ± 114.69 | 81.6 ± 113.85 |
| Hop extract group | 48.1 ± 42.64 | 37.6 ± 29.2 | 35.8 ± 27.47* | 39 ± 29.29 |

As shown in Table 7, as for LDH, no change was observed in the placebo group, whereas a tendency of decrease was observed in the hop extract group and the difference was significant after 8 weeks of ingestion.

TABLE 7

| LDH | IU/l | | | |
|---|---|---|---|---|
| Ingestion (weeks) | 0 | 4 | 8 | 12 |
| Placebo group | 251.7 ± 124.83 | 289.9 ± 193.85 | 275.9 ± 160.13 | 258.6 ± 138.05 |

TABLE 7 (continued)

| LDH | IU/l | | | |
|---|---|---|---|---|
| Hop extract group | 276.8 ± 140.9 | 261.9 ± 133.04 | 260.9 ± 131.27* | 266.4 ± 132.48 |

Example 2: Blood pressure lowering activity of hop extract in spontaneously hypertensive rats

The hop extract (1g/kg) was orally administered to spontaneously hypertensive rats (SHRs) and blood pressure lowering activity was measured. Further, distilled water was used as a control. Four animals each were used in both experimental and control groups and the blood pressure lowering activity was measured as follows. The sample or the control water was orally given to 27-week-old male SHRs (Charles River) into the stomach using a sonde. The temperature was maintained at 38°C for 10 to 20 minutes at a specified interval and systolic blood pressure was measured using a non-invasive blood pressure measuring device (Softron, BP-98A). The results are shown in Table 8. No blood pressure change was observed in the control group, whereas blood pressure started to decrease 4 hours after the administration in the experimental group, the blood pressure lowering activity reached the maximum (-14.0 mmHg as a change in blood pressure) 10 hours after the administration and the blood pressure returned to the starting level 24 hours after the administration,

TABLE 8

| | | Change in systolic blood pressure (mmHg) | | | | | | |
|---|---|---|---|---|---|---|---|---|
| Time after administration (hr) | | 0 | 2 | 4 | 6 | 8 | 10 | 24 |
| Control | Distilled water | 0.0 | 1.1 | 4.6 | 0.1 | -2.1 | -3.8 | 6.0 |
| Experimental | Hop extract | 0.0 | 0.2 | -4.2 | -3.9 | -6.9 | -14.1 | -2.4 |

Example 3: Blood pressure lowering activity of isohumulones in spontaneously hypertensive rats

The isohumulones preparation purified in Reference Example 1 (48% isohumulone, 37% isocohumulone, and 15% isoadhumulone; 83. 8 mg/kg) was orally administered to SHRs and the blood pressure lowering activity was measured. Further, distilled water was used as a control. Four animals each were used in both experimental and control groups and the blood lowering activity was measured in the same manner as described in Example 2, except that 22-week-old SHRs were used. The results are shown in Table 9. No change in blood pressure was observed in the control group, whereas in the experimental group, the blood pressure decreased 4 hours and 10 hours after the administration, the maximum blood pressure lowering activity (-17.4 mmHg as a change in blood pressure) was exhibited 10 hours after the administration and the blood pressure returned to the starting level 24 hours after the administration.

TABLE 9

| | | Change in systolic blood pressure (mmHg) | | | | | | |
|---|---|---|---|---|---|---|---|---|
| Time after administration (hr) | | 0 | 2 | 4 | 6 | 8 | 10 | 24 |
| Control | Distilled water | 0.0 | 4.8 | -1.8 | -1.8 | -4.4 | -5.2 | -5.5 |
| Experimental | Isohumulones | 0.0 | 4.1 | -7.8 | 5.8 | -1.7 | -17.4 | -1.8 |

Example 4: Vasorelaxant effect of isohumulones

Five male Wister rats were killed by drawing blood under anaesthetization with diethyl ether and their thoracic aortas were excised and cut into rings with a length of about 3 mm to prepare ring-shaped vascular specimens. After removing the endothelium, the specimens were hung down into an organ bath filled with 10 ml of Krebs-Ringer solution (containing 112 mM NaCl, 4.7 mM KCl, 2. 2 mM $CaCl_2$, 25 mM $NaHCO_3$, 1. 2 mM $MgCl_2$, 1.2 mM $KH_2PO_4$, 14 mM glucose). The solution in the bath was heated to 37°C and gassed with a mixture of 95% $O_2$ and 5% $CO_2$. The specimens were loaded with 1.5 g of static tension and allowed to stand for 120 minutes. Then, the Krebs-Ringer solution in the bath was replaced with a highly concentrated KCl solution (80 mM) or a Krebs-Ringer solution containing $10^{-6}$ M phenylephrine to contract the specimens in advance. After confirming the tension of the specimens being stabilized, the isohumulone solution purified in Reference Example 1 (containing 48% isohumulone, 37% isocohumulone, and 15% isoadhumulone) or its vehicle (ethanol solution) was accumulatively added. The tension of each specimen was measured by a transducer. The relaxant activity of the substances added was expressed as a percentage relative to the relaxation response by $10^{-4}$ M papavarine added at the end of each experiment.

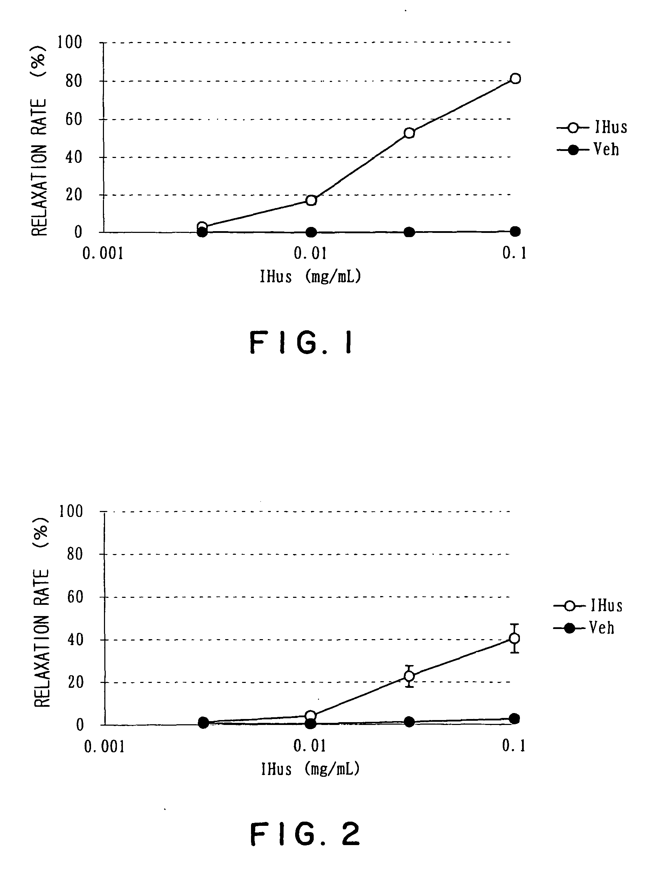

The isohumulones (0.003 to 0.1 mg/ml) relaxed concentration-dependently the rat aortic specimens which were contracted with 80 mM KCl and phenylephrine ($10^{-6}$ M) (Figures 1 and 2).

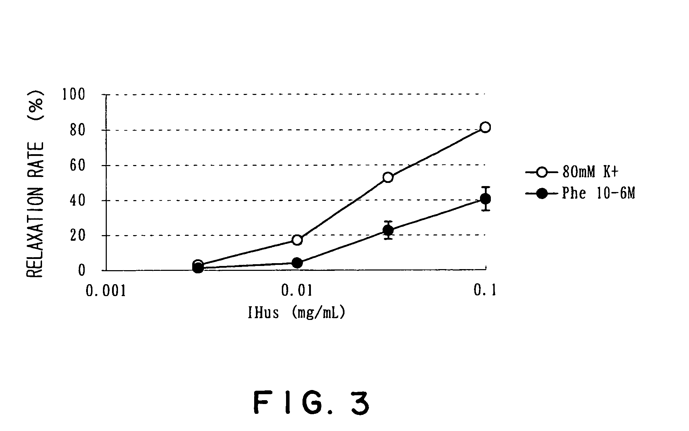

Further, the vasorelaxant reaction by the isohumulones was stronger in 80 mM KCl relaxation specimens than in the phenylephrine relaxation specimens (Figure 3).

Example 5: Improvement of vascular endothelial function by hop extract in human subjects

Ten healthy male and female adults (with an average age of 27.6) were selected as test subjects from whom agreement to participate in this experiment was obtained prior to the start of the experiment in view of the ethical consideration.

The vascular endothelial function was separately measured on each of the same subjects on two different days upon ingestion of the hop extract and upon ingestion of the placebo. Two kinds of soft capsules, one for placebo and one for hop extract were prepared according to an ordinary method. The capsule for hop extract contains 28.3 mg of the hop extract per capsule.

All subjects were fasted and given purified water only as a liquid from 9 pm the day before the experiment. In the following morning, the flow mediated dilatation (FMD) was measured before, 30 minutes after, and 2 hours after administering the soft capsules, one capsule per 10 kg body weight, using an ultrasound echo device (HDI5000, ATL ULTRASOUND Inc.) by a physician. The flow mediated dilatation expressed as %FMD was calculated according to an ordinary method. Briefly, after measuring the diameter of blood vessels at rest, the upper arm was avascularized for 5 minutes at 200 mmHg or higher using a manchette for blood pressure measurement, and then after releasing, the maximum vessel diameter was measured. The %FMD was calculated using to the following formula:

$$\%FMD = \{(maximum\ vessel\ diameter - vessel\ diameter\ at\ rest)/vessel$$

$$diameter\ at\ rest\} \times 100$$

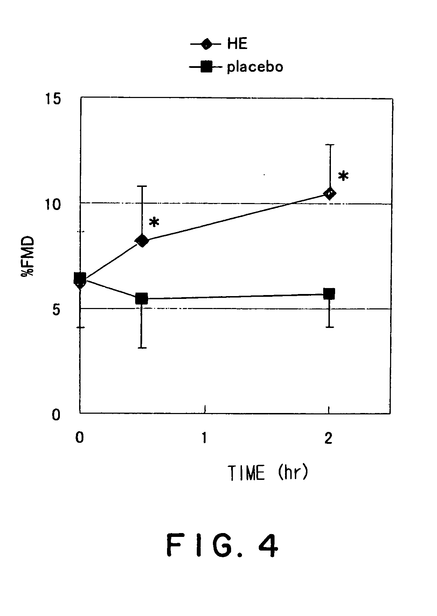

The results are shown in Figure 4. As evident from the results, a significant increase in %FMD was observed 30 minutes and 2 hours after the ingestion of the hop extract, whereas absolutely no change was observed upon ingesting the placebo. From these results, it is understood that the vascular endothelial function was quickly improved by the ingestion of the hop extract.

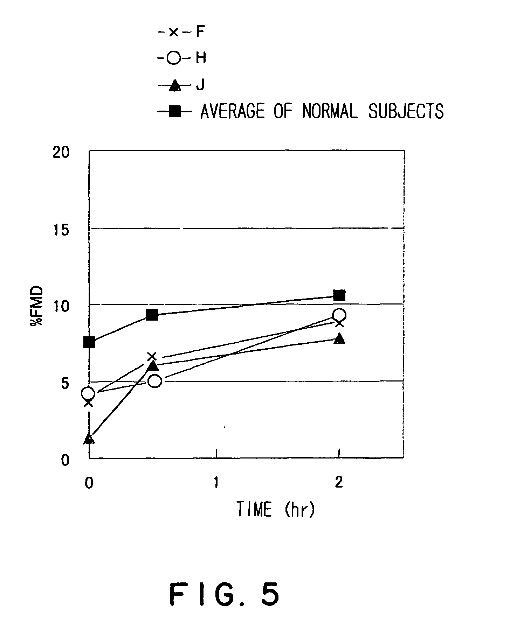

The %FMD of healthy subjects was about 6% or more: the measurement results revealed that 3 subjects in this experiment (F, H, and J) had declined vascular endothelial function. Accordingly, the effect of the hop extract was compared between generally healthy subjects with %FMD of 5 or more before soft capsule ingestion and subjects with lower %FMD and having declined vascular endothelial function, as shown in Figure 5. As evident from the result, the hop extract improved vascular endothelial function of the healthy subjects. Further, the extract also ameliorated vascular endothelial function of the subjects having declined vascular endothelial function to a normal level. Therefore, it is understood that the hop extract has an effect not only to improve vascular endothelial function of healthy subjects but also to ameliorate vascular endothelial function of subjects having declined function to a normal level.

**Claims**

1. A composition for lowering blood pressure, comprising isohumulones or a hop extract and/or an isomerized hop extract as an active ingredient.

2. A composition for preventing, treating or ameliorating hypertension, comprising isohumulones, or a hop extract and/or an isomerized hop extract as an active ingredient.

3. A composition for improving or ameliorating vascular flexibility, comprising isohumulones, or a hop extract and/or an isomerized hop extract as an active ingredient.

4. A composition for improving or ameliorating vascular endothelial function, comprising isohumulones, or a hop extract and/or an isomerized hop extract as an active ingredient.

5. A composition for vascular dilatation or blood flow acceleration, comprising isohumulones, or a hop extract and/or an isomerized hop extract as an active ingredient.

6. The composition according to any one of claims 1 to 5, wherein the isohumulones are selected from the group consisting of isohumulone, isoadhumulone, isocohumulones, and combinations thereof.

7. The composition according to any one of claims 1 to 6, wherein said composition is provided in the form of a food.

8. The composition according to claim 7, wherein the food is a drink.

9. The composition according to claim 8, wherein the drink is a non-alcohol drink.

10. The composition according to claim 7, 8 or 9, wherein the food is a health food, a functional food, a food for specific health use, or a food for patients.

11. The composition according to any one of claims 1 to 6, wherein said composition is provided in the form of a medicine.

12. A method of lowering blood pressure, comprising administering to a mammal isohumulones, or a hop extract and/ or an isomerized hop extract.

13. A method of preventing, treating or ameliorating hypertension, comprising administering to a mammal isohumulones, or a hop extract and/or an isomerized hop extract.

14. A method of improving or ameliorating vascular flexibility, comprising administering to a mammal isohumulones, or a hop extract and/or an isomerized hop extract.

15. A method of improving or ameliorating vascular endothelial function, comprising administering to a mammal isohumulones, or a hop extract and/or an isomerized hop extract.

16. A method of dilating blood vessels or accelerating blood flow, comprising administering to a mammal isohumulones, or a hop extract and/or an isomerized hop extract.

17. The method according to any one of claims 12 to 16, wherein the isohumulones are selected from the group consisting of isohumulone, isoadhumulone, isocohumulones, and combinations thereof.

18. Use of isohumulones, or a hop extract and/or an isomerized hop extract for the manufacture of a composition for lowering blood pressure.

19. Use of isohumulones, or a hop extract and/or an isomerized hop extract for the manufacture of a composition for preventing, treating, or ameliorating hypertension.

20. Use of isohumulones, or a hop extract and/or an isomerized hop extract for the manufacture of a composition for improving and ameliorating vascular flexibility.

21. Use of isohumulones, or a hop extract and/or an isomerized hop extract for the manufacture of a composition for improving and ameliorating vascular endothelial function.

22. Use of isohumulones, or a hop extract and/or an isomerized hop extract for the manufacture of a composition for dilating blood vessels or accelerating blood blow.

23. The use according to any one of claims 18 to 22, wherein the isohumulones are selected from the group consisting of isohumulone, isoadhumulone, isocohumulones, and combinations thereof.

24. A food for lowering blood pressure, comprising isohumulones, or a hop extract and/or an isomerized hop extract.

25. A food for preventing, treating, or ameliorating hypertension, comprising isohumulones, or a hop extract and/or an isomerized hop extract.

26. A food for improving or ameliorating vascular flexibility, comprising isohumulones, or a hop extract and/or an isomerized hop extract.

**27.** A food for improving or ameliorating vascular endothelial function, comprising isohumulones, or a hop extract and/or an isomerized hop extract.

**28.** Food for dilating blood vessels or accelerating blood flow, comprising isohumulones, or a hop extract and/or an isomerized hop extract.

**29.** The food according to any one of claims 24 to 28, wherein the isohumulones are selected from the group consisting of isohumulone, isoadhumulone, isocohumulones, and combinations thereof.

**30.** The food according to any one of claims 24 to 29, wherein the isohumulones are provided daily in a range of 30 mg to 6000 mg on the basis of an amount of isohumulones.

**31.** The food according to any one of claims 24 to 30, which is in the form of a drink.

**32.** The food according to claim 31, wherein the drink is a non-alcohol drink.

**33.** The food according to any one of claims 24 to 32, which is a health food, a functional food, a food for specific health use, or a food for patients.

**34.** A non-alcohol drink comprising isohumulones, or a hop extract and/or an isomerized hop extract, wherein the isohumulones, or a hop extract and/or an isomerized hop extract are provided daily in a range of 30 mg to 6000 mg on the basis of an amount of isohumulones.

**35.** The non-alcohol drink according to claim 34, wherein the isohumulones, or a hop extract and/or an isomerized hop extract are provided daily in a range of 60 mg to 3000 mg on the basis of an amount of isohumulones.

**36.** The non-alcohol drink according to claim 34 or 35, which is a tea drink.

FIG. 1

FIG. 2

F I G. 3

FIG. 4

FIG. 5

**INTERNATIONAL SEARCH REPORT**

| International application No. |
| --- |
| PCT/JP2004/000324 |

A. CLASSIFICATION OF SUBJECT MATTER
    Int.Cl⁷ A61K31/122, 35/78, A61P9/00, 9/08, 9/12, A23L1/30

According to International Patent Classification (IPC) or to both national classification and IPC

B.  FIELDS SEARCHED

Minimum documentation searched (classification system followed by classification symbols)
    Int.Cl⁷ A61K31/122, 35/78, A61P9/00, 9/08, 9/12, A23L1/30

Electronic data base consulted during the international search (name of data base and, where practicable, search terms used)
    CA(STN), MEDLINE(STN), BIOSIS(STN), JICST(JOIS)

C.  DOCUMENTS CONSIDERED TO BE RELEVANT

| Category* | Citation of document, with indication, where appropriate, of the relevant passages | Relevant to claim No. |
| --- | --- | --- |
| P,X | JP 2003-226640 A  (Akita-ken), 12 August, 2003 (12.08.03), Claims 1 to 3; Par. Nos. [0002], [0009] (Family: none) | 1-2,6-11, 18-19,23-25, 29-36 |
| Y | FUJIWARA et al., "Beer Nigami Seibun no PPARγ Kasseika Sayo", The Japanese Society of Nutrition and Food Science Sokai Koen Yoshishu, 2002, Vol.56th, page 161 | 1-11,18-36 |
| Y | HOSONO et al., "Beer Nigami Seibun no Shishitsu Taisha Kaizen Koka", The Japanese Society of Nutrition and Food Science Sokai Koen Yoshishu, 2002, Vol.56th, page 354 | 1-11,18-36 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 22 March, 2004 (22.03.04) | 06 April, 2004 (06.04.04) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

| | INTERNATIONAL SEARCH REPORT | International application No. |
|---|---|---|
| | | PCT/JP2004/000324 |

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 2002-332266 A  (Sankyo Co., Ltd.),<br>22 November, 2002 (22.11.02),<br>Claim 19<br>& EP 1277729 A1 | 1-2,6-11,<br>18-19,23-25,<br>29-36 |
| Y | WO 02/051820 A1  (ONO PHARMACEUTICAL CO., LTD.),<br>04 July, 2002 (04.07.02),<br>Column 3, lines 15 to 24<br>& EP 1354879 A1 | 1-2,6-11,<br>18-19,23-25,<br>29-36 |
| Y | SAITO et al., "PPAR to Kekkan", Egaku no Ayumi,<br>2001, Vol.198, No.11, pages 766 to 767,<br>particularly, page 766, left column, line 19 | 3-11,20-23,<br>26-36 |

Form PCT/ISA/210 (continuation of second sheet) (January 2004)

| **INTERNATIONAL SEARCH REPORT** | International application No. |
|---|---|
| | PCT/JP2004/000324 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 12 to 17
   because they relate to subject matter not required to be searched by this Authority, namely:
   It pertains to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest.

☐ No protest accompanied the payment of additional search fees.

Form PCT/ISA/210 (continuation of first sheet (2)) (January 2004)